# EUROPEAN PATENT APPLICATION

(11) **EP 1 588 663 A1**
(43) Date of publication of application: **26.10.2005**
(21) Application number: 05008588.5
(22) Date of filing: 20.04.2005
(51) Int. Cl.: A61B 5/117, G06K 9/00

(54) **Iris recognition apparatus with different states of illumination**

(30) Priority: 22.04.2004 JP 2004126438
(71) Applicant: MATSUSHITA ELECTRIC INDUSTRIAL CO., LTD., Kadoma-shi, Osaka 571-8501 (JP)
(72) Inventor: Ikoma, Ken, Yokohama-shi Kanagawa-ken 225-0023 (JP); Nakaigawa, Tomoyoshi, Yokohama-shi Kanagawa-ken 244-0802 (JP)
(74) Representative: Pautex Schneider, Nicole Véronique

(57) **Abstract**

An eye image capturing apparatus includes a capturing unit which captures an eye image of a person to be authenticated, a plurality of illuminating devices which illuminate the eye of the person to be authenticated, and are disposed so as to have different distances with respect to an optical axis of the capturing unit, and a control unit which controls sequentially illuminations of the illuminating devices to capture the eye image at each timing of the illuminations.

## Description

### BACKGROUND OF THE INVENTION

The present invention relates to an eye image capturing apparatus for use in an authentication system for implementing a personal authentication through the pattern of a person's retina or iris and, more particularly, to an eye image capturing apparatus with illuminating devices which has a function to illuminate the eye as an object to be captured.

For example, in an iris authentication system provided at an entrance to a room for determining on the eligibility for entrance to the room, an image of the eye of a person who wishes to enter the room is captured for authentication by an eye image capturing apparatus, and an iris image portion is cut out to extract a quantity of characteristics or unique features to the person. Then, the feature quantity so extracted is compared and collated with a feature quantity which was extracted from the iris image of a registered person who has been authorized for entrance to the room and which has been registered in a database, whereby whether or not the person under authentication coincides with the registered person is determined.

When a feature quantity for use for comparison and collation like this is extracted from an iris image, in the event that an image of the eye obtained from a person to be authenticated is not in a good condition, an authentication process cannot be performed with high accuracy. Due to this, an illuminating device is added to an eye image capturing apparatus so that the eye, which is an object to be captured, is illuminated. In the case of iris images, since clear images can be captured by illuminating the eye with infrared light, an infrared illuminating device is added.

When an image of the eye of a person to be authenticated is captured by the eye image capturing apparatus while illuminating the iris of the person with the illuminating device, in the event that reflected light resulting when illuminating light is reflected on the eyeball enters the capturing apparatus, the image of the illuminating device is caused to be reflected in the image of the iris, and the image of the iris which shows the image of the illuminating device reflected therein becomes inappropriate as an iris image for use for authentication. In particular, when a person to be authenticated wears a pair of glasses, possibility that the image of the illuminating device is superposed on the iris image is increased, which causes a problem.

Due to this, conventionally, as described in JP-A-2001-167284, whether or not a reflected portion of illuminating light exists in a captured iris image is detected through the area of a region with high luminance in the captured iris image. In addition, in order for reflected light not to enter the capturing apparatus even in the event that the illuminating light is reflected on the pair of glasses, illuminating light is emitted largely obliquely with respect to the eye, so that reflected light does not enter the capturing apparatus.

When illuminating light is made to be emitted largely obliquely, however, a position where the illuminating device needs to be spaced apart from an optical axis of the eye image capturing apparatus, and this causes a problem that the lateral width of the overall eye image capturing apparatus has to be increased, and hence, the eye image capturing apparatus cannot be made smaller in size. In addition, the iris image can be captured in a better condition as illuminating light is emitted more square thereto, whereas as the illuminating light is emitted more obliquely with respect to the eye, a possibility is increased that a problem is caused that a dark portion is created in the iris, whereby the quality of a captured image is deteriorated.

### SUMMARY OF THE INVENTION

An object of the invention is to provide an eye image capturing apparatus with illuminating devices which can realize the miniaturization of the apparatus and can capture an eye image of high quality.

In order to achieve the above object, according to the present invention, there is provided an eye image capturing apparatus, comprising:
a capturing unit which captures an eye image of a person to be authenticated;
a plurality of illuminating devices which illuminate the eye of the person to be authenticated, and are disposed so as to have different distances with respect to an optical axis of the capturing unit; and
a control unit which controls sequentially illuminations of the illuminating devices to capture the eye image at each timing of the illuminations.

### [NEW]

Preferably, the illuminating devices are turned on in the order of proximity to the optical axis for the sequentially illuminations.

According to this configuration, since the eye image can be captured which is illuminated as square to the person to be authenticated as possible, a captured image of high quality can be obtained.

Preferably, wherein the illuminating devices are arranged so that illuminating light directions of the illuminating devices are oblique with respect to the optical axis of the capturing unit.

According to this configuration, the direction of the eye of the person to be authenticated can be illuminated efficiently.

Preferably, the illuminating devices are provided in pluralities of rows and columns.

According to this configuration, a possibility of selection of an illuminating device is increased in which illuminating light is reflected in the eye image of the person to be authenticated.

Preferably, the illuminating devices includes an infrared-light illuminating device for illuminating the eye of the person with infrared light, and a visible-light illuminating device provided adjacent to the infrared-light illuminating device for illuminating the eye of the person with visible light. The infrared illuminating device and the visible-light illuminating device are illuminated at the same time.

According to this configuration, the person to be authenticated can alter the orientation of his or her own face and the pair of glasses he or she wears so as to adjust them to prevent the reflection of the illuminating infrared light in the eye image.

Preferably, the eye image capturing apparatus further includes a half mirror which is provided on the optical axis of the capturing unit for reflecting the eye of the person, and an adjustment unit on which the capturing unit, the infrared-light illuminating device, the visible-light illuminating device and the half mirror are mounted. The adjustment unit is rotatably provided to adjust a direction of the optical axis.

According to this configuration, the person to be authenticated can alter the orientation of his or her own face and the pair of glasses he or she wears so as to adjust them to prevent the reflection of the illuminating infrared light in the eye image.

### [NEW]

Preferably, the control unit analyzes the image of the eye obtained by the capturing unit each time of capturing the image of the eye.

Preferably, the control unit analyzes the images of the eye obtained by the capturing unit after capturing all of the images of the eye at timings of the illuminations.

Preferably, the control unit determines that whether or not reflected light exists in the eye image when the control unit analyzes the images of the eye obtained by the capturing unit. The control unit selects the eye image of the person to be authenticated in which the reflected light of illumination light emitted from the illuminating device is not superposed on the eye image.

According to this configuration, an iris image of high quality can be captured within a short period of time.

Preferably, when reflected light exists in the eye images, the control unit extracts a plurality of partial eye images where the reflected light does not exist from the eye images. The control unit synthetically incorporates the partial eye images into an eye image in which the reflected light does not exist.

According to this configuration, the number of times of capturing the eye image can be reduced, whereby the shortening of time during which the person to be authenticated has to wait for completion of authentication.

According to the invention, an eye image of high quality, which is appropriate to authentication, can be captured with ease.

### BRIEF DESCRIPTION OF THE DRAWINGS

The above objects and advantages of the present invention will become more apparent by describing in detail preferred exemplary embodiments thereof with reference to the accompanying drawings, wherein:
Fig. 1A is a front view of an eye capturing apparatus according to a first embodiment of the invention, and Fig. 1 B is a right side view of the eye image capturing apparatus according to the first embodiment of the invention;
Fig. 2 is an exemplary front view of a camera accommodating portion according to the first embodiment of the invention;
Fig. 3 is a front view of the eye which explains the first embodiment of the invention;
Fig. 4 is a flowchart illustrating a capturing procedure of the eye image capturing apparatus according to the first embodiment of the invention;
Fig. 5 is an explanatory drawing which explains an illuminating order according to the first embodiment of the invention;
Fig. 6 is an explanatory drawing which explains illuminated states according to the first embodiment of the invention;
Fig. 7 is a flowchart illustrating a capturing procedure of an eye image capturing apparatus according to a second embodiment of the invention;
Fig. 8 is an exemplary front view of a camera accommodating portion according to a third embodiment of the invention; and
Fig. 9A is an explanatory drawing which explains illumination according to the third embodiment of the invention, and Fig. 9B is an explanatory drawing which explains the adjustment of the angle of the face according to the third embodiment of the invention.

### DETAILED DESCRIPTION OF THE PREFERRED EMBODIMENTS

Embodiments of the invention will be described below by reference to the accompanying drawings.

### (First Embodiment)

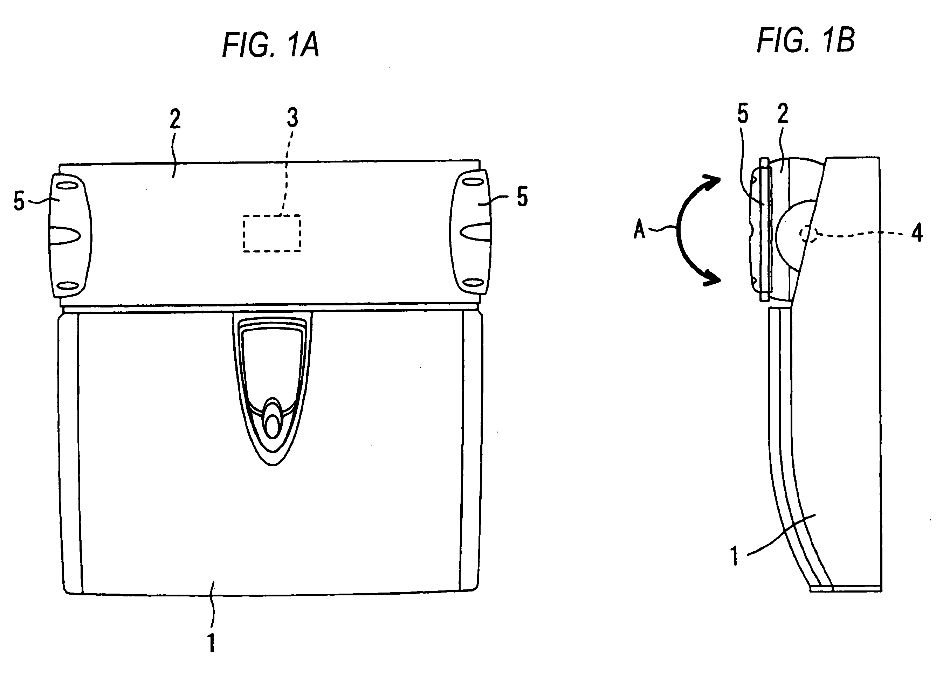

Fig. 1A is a front view of an eye image capturing apparatus according to a first embodiment of the invention, and Fig. 1 B is a right side view thereof. The eye image capturing apparatus of the embodiment is constructed to capture an iris image of a person to be authenticated, and a camera accommodating portion 2 which accommodates therein a capturing unit (hereinafter, referred to as a camera) is provided at an upper portion of a main body case 1.

A half mirror 3 is mounted in a front central portion of the camera accommodating portion 2, and a lens (not shown) of an iris capturing camera, not shown, is placed on a back side of the half mirror 3.

As indicated by a double-headed arrow A in Fig. 1 B, the camera accommodating portion 2 is supported about an axis 4 in such a manner as to be rotatable with respect to the main body case 1, and a person to be authenticated, who is standing in front of the main body case 1 of the eye image capturing apparatus, rotates with his or her own hands grips 5 provided at both end portions of the camera accommodating portion 2 so as to adjust the apparatus such that his or her own right or left eye is reflected in the half mirror 3.

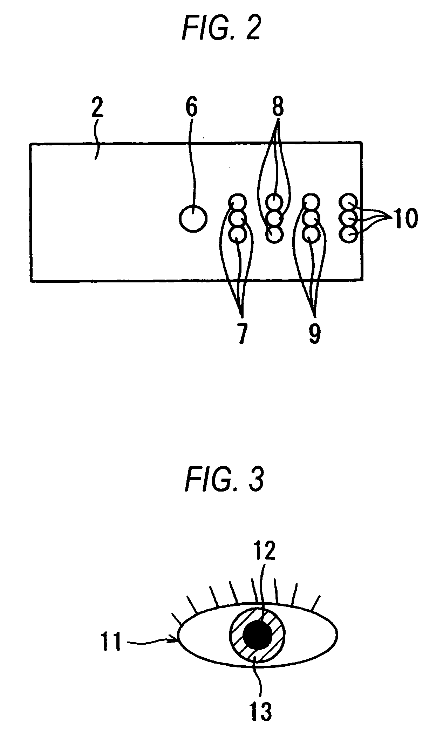

Fig. 2 is an exemplary front view of the camera accommodating portion 2. As is described above, the lens 6 of the iris capturing camera is provided in the central portion of the camera accommodating portion 2, and a plurality of light emitting diodes which emit infrared rays are provided on one side thereof. In total, 12 light emitting diodes are arranged in 3 rows and 4 columns on the illustrated camera accommodating portion 2 in such a manner that as viewed from a side proximate to the lens 6, 3 light emitting diodes 7 are arranged in a first column, 3 light emitting diodes 8 in a second column, 3 light emitting diodes in a third column and 3 light emitting diodes 10 in a fourth column. The illuminating directions of illuminating light from the light emitting diodes in the respective columns 7, 8, 9, 10 are inclined so as to be oriented towards an object to be captured (the eye of the person to be authenticated) in front of the lens 6.

Fig. 3 is a front view of the eye. There exists a pupil 12 at a central portion of the eye 11, and a ring-like iris portion 13 exists in such a manner as to surround the pupil 12. the iris capturing camera of the eye image capturing apparatus is made to capture an image of the eye 11, and an arithmetic processing unit provided on the eye image capturing apparatus cuts an iris image out of the captured eye image, further converts the image so cut out into a code so as to represent a feature quantity in code and sends the coded feature quantity to an iris authenticating unit.

In the event that the illuminating light emitting diode is reflected in a ring-like iris image portion, when the iris image capturing camera captures an image of the eye, namely, in the event that the person to be authenticated wears a pair of glasses and reflected light resulting when illuminating light is reflected on the pair of glasses enters the lens 6 to thereby be superposed on the iris portion of the person to be authenticated, the resulting iris image becomes eclipsed by a portion of high luminance.

Then, in this embodiment, the diode whose illuminating light is not reflected in the iris image is sought for from, in total, the twelve light emitting diodes 7, 8, 9, 10, so as to capture an iris mage in a good condition.

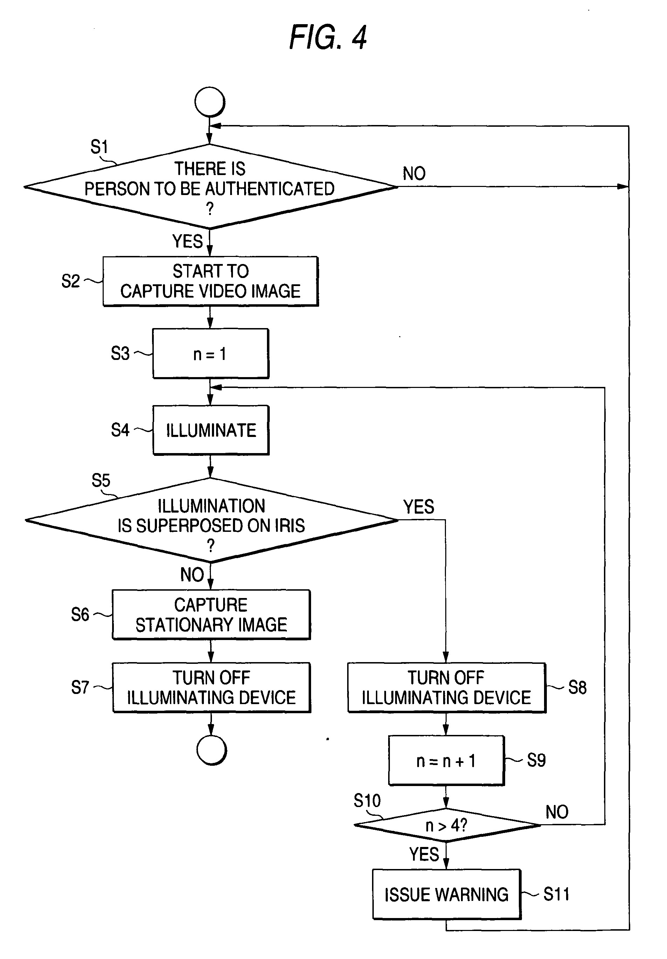

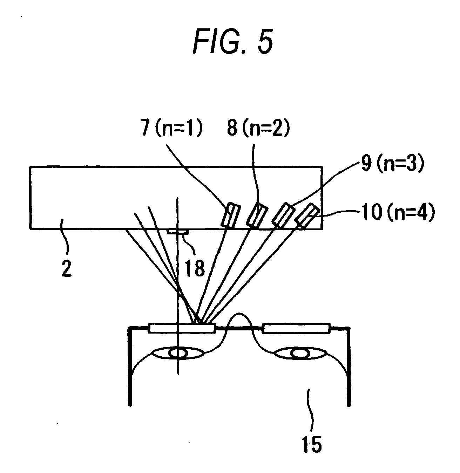

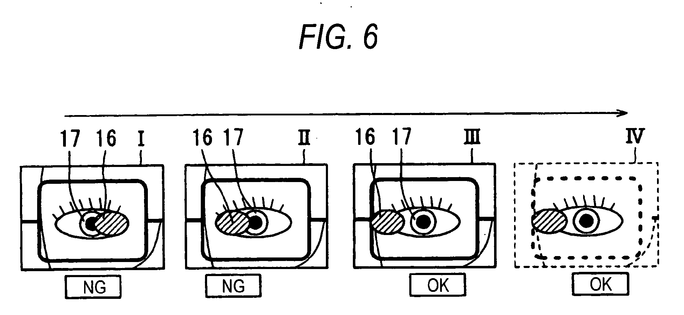

Fig. 4 is a flowchart illustrating a procedure of an eye image capturing process that is executed by a control unit of the eye image capturing apparatus, and Figs. 5, 6 are drawings which explain the illuminating state of each light emitting diode. In this embodiment, a case will be described in which only four diodes in the first row of the diodes arranged in 3 rows and 4 columns are provided. In addition, light emitting diode numbers for the light emitting diodes 7, 8, 9, 10 are indicated as n=1, n=2, n=3, n=4, respectively.

When a person to be authenticated stands in front of the main body case 1, the eye image capturing apparatus, which has detected (step S1) this fact with, for example, a sensor, drives solid body capturing devices such as CCD (charge coupled device) or CMOS of the iris capturing camera and starts a process of taking in the object to be captured as a moving or video image or taking in the image of the object to be captured in a predetermined cycle (step S2).

Next, letting the number n of the light emitting diodes that are used as the illuminating device be n=1 (step S3), the light emitting diodes of that number n are illuminated (step S4). In this case, since the number n=1 indicates the light emitting diodes 7 which are closest to the lens 6 or the optical axis of the iris capturing camera, the light emitting diodes 7 are illuminated through an instruction from an illumination control unit of the eye image capturing apparatus.

Next, the video image captured under the illuminating light emitted from the light emitting diodes is analyzed to determine whether or not the high-luminance area is superposed on the iris image (step S5). If the high-luminance area is not superposed on the iris image, since the iris image captured is in a good condition, the iris capturing camera captures an image of the eye of the person to be authenticated as a stationary image (step S6), and the light emitting diodes, which are being illuminated, are turned off (step S7), whereby the eye image capturing process ends. Thereafter, an iris image cutting out process and a feature quantity coding process follow.

As shown in Fig. 5, the light emitting diodes 7 (n=1) are illuminated to illuminate the direction of the eye of the person 15 to be authenticated, and as shown in an image state I in Fig. 6, if it is detected that a high-luminance area 16 due to the illuminating light exists in an output image from the solid body capturing device and that this high-luminance area 16 is superposed on an iris image 17, the results of the determination made in step S5 is denied.

In this case, the light emitting diodes, which are being illuminated, are turned off (step S8). Next, let the light emitting diode number n be n=n+1 (step S9), and whether or not the value of n exceeds the number of light emitting diodes (in this embodiment, since there exist the four diode numbers, 7, 8, 9, 10, n=4) is determined (step S10). If the value of n does not exceed the number of light emitting numbers, the flow returns to step S4 to illuminate the light emitting diodes of that number n.

From this, the light emitting diodes 8 are illuminated after the light emitting diodes 7, and as shown in an image state II in Fig. 6, whether or not a high-luminance are is superposed on an iris image is determined (step S5). If the high-luminance are 16 is also superposed on the iris image 17 even under the illumination by the light emitting diodes 8, the next light emitting diodes 9 are illuminated in a similar manner.

Here, as shown in an image state III in Fig. 6, if it is detected that a high-luminance area 16 deviates from an iris image 17, another stationary image is captured (step S6), and the capturing process is completed, whereby a state IV in Fig. 6 or a state where the light emitting diodes 10 are illuminated is no more required.

In the related case, in consideration of the shapes and variations of pairs of glasses wore on the person, the illuminating device is disposed at the position which is located farthest from the optical axis of the camera in order to prevent the illuminating device from being reflected in the iris image, and due to this, the inclination angle of the illuminating light with respect to the iris portion becomes large, posing one of the causes for the deterioration of the quality of the iris image. Since only the light emitting diodes 10 in Fig. 5 are provided in the related apparatus, even when the bare eye is attempted to be illuminated, the bare eye has to be illuminated with the illuminating light which is largely inclined, thereby making it impossible to obtain an iris image of good quality.

On the contrary to this, in the embodiment of the invention, since the iris image can be captured while being illuminated using the light emitting diodes which are arranged inwards of the outermost light emitting diodes 10 or whose illuminating light has a smaller inclination angle with respect to the iris portion, the capturing of the iris image of high quality can be realized.

Returning to Fig. 4, if the light emitting diode number n for the light emitting diodes to be illuminated advances from the light emitting diodes 7, 8, 9 to 10, and the high-luminance area 16 continues to be superposed on the iris image 17 under illumination by any of those light emitting diodes, next is n>4 (step S10). In this event, the person to be authenticated is advised to incline his or her glasses, for example, (step S11), the flow returns to the initial step S1.

In the embodiment that has been described heretofore, while the case has been described where the four light emitting diodes in the single horizontal row are provided, when the high-luminance area 16 is superposed on the iris image 17, the superposition of the high-luminance area 16 on the iris image 17 can be prevented by altering the vertical position of the light emitting diodes which are provided in three rows as shown in Fig. 2. Namely, as the light emitting diodes to be illuminated, the innermost three light emitting diodes, which are closest to the lens 6, are sequentially illuminated in the vertical direction, and the next innermost three light emitting diodes are sequentially illuminated in the vertical direction, and this procedure may be repeated.

### (Second Embodiment)

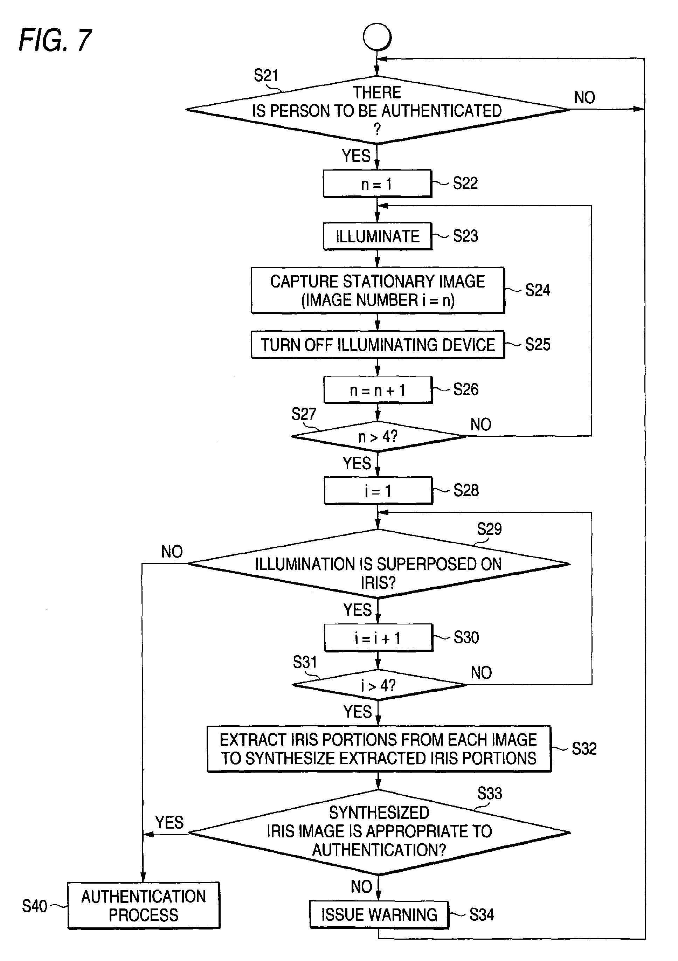

Fig. 7 is a flowchart illustrating a procedure of an eye image capturing process which is executed by a control unit of an eye image capturing apparatus according to a second embodiment of the invention. Note that light emitting number n used in the flowchart below remains the same as that of the first embodiment.

Firstly, a person to be authenticated waits for an eye image capturing apparatus to be brought in front of him or her (step S21), and the light emitting diode number n is let be n=1 when the person to be authenticated arrives (step S22). Then, light emitting diodes whose light emitting number is n=1 are illuminated (step S23) so as to capture a stationary image of the iris (step S24). Let the stationary image number i of the stationary image obtained then be i=n. Namely, the number of the stationary image obtained by illuminating the light emitting diodes of n=1 becomes i=1.

The light emitting diodes of n=1 are turned off after the stationary image has been captured (step S25), and next, let the light emitting diode number n be n+1 or n=n+1 (step S26). Next, whether or not n>4 is determined (step S27), and if negative (No), the flow returns to step S23.

Namely, in this embodiment, stationary images are continuously captured while illuminating the light emitting diodes sequentially, whereby in this embodiment, four stationary images (eye images) illuminated differently are continuously captured in advance and are then stored in a memory, not shown.

When eye images in the same number as that of illuminating light emitting diodes have been captured, the determination in the step S27 becomes affirmative (YES), and next, an eye image of the image number i=1 is read from the memory (step S28). Then, whether or not the illuminating light is superposed on an iris portion of the eye image of the image number i=1 is determined (step S29). If the superposition is denied, the eye image of the image number i=1 is handed over to an authentication process S40 that will be performed in a later step, and the eye image capturing process is completed.

If the illumination is superposed on the iris portion of the eye image of the image number i=1 (the result of the determination is YES), next, an eye image of the image number i=i+1 is fetched from the memory (step S30), and whether or not the image number i is larger than the number of eye images (in the aforesaid embodiment, "4") is determined (step S31). If the image number i is equal to or smaller than the number of eye images, the flow returns to the step S29, whereby whether or not the illumination is superposed on an iris portion of an eye image of the image number i=2 is determined, and if the superposition is denied, the eye image of the image number i=2 is handed over to the authentication process S40 that will be performed in the later step.

Thus, the four eye images are determined sequentially in a manner as done above so as to select the eye image in which no illumination is superposed on the iris portion, and the eye image so selected is handed over to the authentication process S40. However, in the event that all the four eye images are such that the illumination is superposed on the iris portion, the result of the determination in the step S31 becomes affirmative (YES).

In this embodiment, in the event that the superposition of the illumination on the iris portion is true in all the eye images, next, a partial iris image where there is found no superposition of the illumination thereon is extracted from each eye image, and the partial iris images so extracted are affixed together in such a manner that the superposed portions of the extracted partial iris images are superposed on one another so as to synthesize a single iris image (step S32).

In this embodiment, since the four eye images are first captured continuously within a short period of time, and since the person to be authenticated is in a stationary state during the short period of time which is necessary for capturing, the difference among the four eye images is just a difference in illuminating position, and the synthesized image of the iris portion coincides with the single captured image. In the event that the illuminations in the respective eye images are superposed on one another, however, an eclipsed area due to the illumination is created also in the synthesized iris image.

Then, next, whether or not the synthesized iris image is an appropriate image to authentication process or whether or not the size of the eclipsed area is equal to or larger than a predetermined level is determined (step S33), and if appropriate to authentication, the synthesized iris image is handed over to the authentication process S40 in the later step and the eye image capturing process procedure is completed. In addition, if the iris image is determined to be inappropriate to the authentication, a warning is outputted to advise the person to be authenticated to incline his or her own glasses (step S34), and the flow returns to the step S21.

According to the embodiment, since the eye images in the number equal to the number of illuminations are first captured continuously by altering the illuminating position, the eye images of high quality can be captured within a short period of time. In addition, even in the event that the illumination is superposed on each eye image, since the iris image is synthesized by affixing together the partial iris images where the illumination is not superposed on the iris portion, the number of times of capturing eye images can be reduced, thereby making it possible to shorten the time when the person to be authenticated has to wait.

### (Third Embodiment)

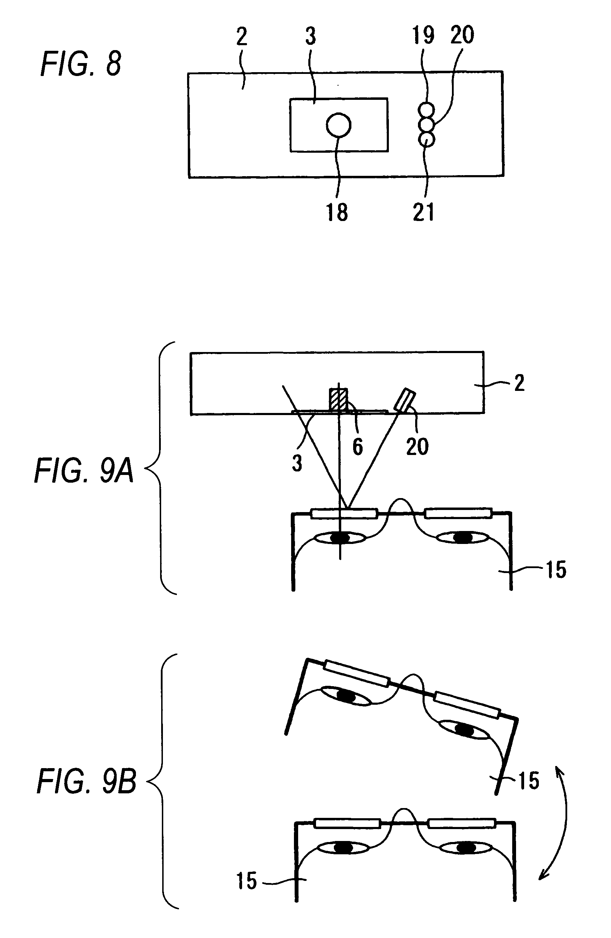

Fig. 8 is an exemplary front view of a camera accommodating portion according to a third embodiment of the invention. In the illustrated embodiment, an eye alignment mark 18 is provided at the center of a half mirror 3 provided at a central portion of the camera accommodating portion 2, and three light emitting diodes 19, 20, 21 are provided at the side of the half mirror 3. The central light emitting diode 20 is made to emit visible light, and the upper and lower light emitting diodes 19, 21 are made to emit infrared rays.

As with the first embodiment, while either of the light emitting diodes 19, 21 is illuminated to capture a stationary image in which a high-luminance area 16 is not superposed on an iris image 17, in the event that the light emitting diode, either the light emitting diode 19 or the light emitting diode 21, is reflected in the iris portion of a person to be authenticated, it will be easiest to ask directly the person to be authenticated to adjust the orientation of his or her own glasses or the angle of his or her face.

However, even in the event that the light emitting diodes 19, 21 are illuminated to emit infrared ray to the eye of the person to be authenticated, since infrared ray is an invisible light, the person to be authenticated himself or herself cannot realize that the light emitting diodes 19, 21 are reflected in his or her iris portion. Then, in this embodiment, when either of the light emitting diodes 19, 21 is illuminated, the visible-light light emitting diode 20, which is provided adjacent to the light emitting diodes 19, 21 is illuminated together.

By adopting this configuration, when reflected light of the visible light is reflected in the eye alignment mark 18 as shown in Fig. 9A, the person to be authenticated 15 can alter the orientation of the glasses or the angle of the face as shown in Fig. 9B so that the visible light is not reflected in the eye alignment mark 18, whereby the high-luminance area due to the infrared ray is allowed to deviate from the iris image, thereby making it possible to capture a good iris image.

Note that while in this embodiment, the three light emitting diodes 19, 20, 21 are disposed adjacent to each other in the vertical direction, the center light emitting diode 20 being made to emit visible light, the plurality of diodes may be arranged as shown in Fig. 2, and the light emitting diodes in the central row are made to emit visible light. Thus, the construction according to the third embodiment may be made to be used in parallel with the construction of the first embodiment.

In addition, while the embodiments that have been described heretofore are described as the iris images are captured, it goes without saying that the configurations of the respective embodiments can be applied to a case where a retina image is captured through the pupil.

The invention provides an advantage that the image quality of an eye image for use for authentication process can be improved by illuminating the eye of a person to be authenticated as square thereto as possible and is effective for use as the eye image capturing apparatus for use for a personal authentication system.

### [NEW]

Although the invention has been illustrated and described for the particular preferred embodiments, it is apparent to a person skilled in the art that various changes and modifications can be made on the basis of the teachings of the invention. It is apparent that such changes and modifications are within the spirit, scope, and intention of the invention as defined by the appended claims.

The present application is based on Japan Patent Application No. 2004-126438 filed on April 22, 2004, the contents of which are incorporated herein for reference.

## Claims

1. An eye image capturing apparatus, comprising:
a capturing unit which captures an eye image of a person to be authenticated;
a plurality of illuminating devices which illuminate the eye of the person to be authenticated, and are disposed so as to have different distances with respect to an optical axis of the capturing unit; and
a control unit which controls sequentially illuminations of the illuminating devices to capture the eye image at each timing of the illuminations.

2. The eye image capturing apparatus as set forth in claim 1, wherein the illuminating devices are turned on in the order of proximity to the optical axis for the sequentially illuminations.

3. The eye image capturing apparatus as set forth in claim 1, wherein the control unit analyzes the image of the eye obtained by the capturing unit each time of capturing the image of the eye.

4. The eye image capturing apparatus as set forth in claim 1, wherein the control unit analyzes the images of the eye obtained by the capturing unit after capturing all of the images of the eye at timings of the illuminations.

5. The eye image capturing apparatus as set forth in claim 1, wherein the illuminating devices are arranged so that illuminating light directions of the illuminating devices are oblique with respect to the optical axis of the capturing unit.

6. The eye image capturing apparatus as set forth in claim 1, wherein the illuminating devices are provided in pluralities of rows and columns.

7. The eye image capturing apparatus as set forth in claim 1, wherein the illuminating devices includes:
an infrared-light illuminating device for illuminating the eye of the person with infrared light; and
a visible-light illuminating device provided adjacent to the infrared-light illuminating device for illuminating the eye of the person with visible light; and
wherein the infrared illuminating device and the visible-light illuminating device are illuminated at the same time.

8. The eye image capturing apparatus as set forth in claim 7, further comprising a half mirror which is provided on the optical axis of the capturing unit for reflecting the eye of the person; and
an adjustment unit on which the capturing unit, the infrared-light illuminating device, the visible-light illuminating device and the half mirror are mounted, and the adjustment unit being rotatably provided to adjust a direction of the optical axis.

9. The eye image capturing apparatus as set forth in claim 4, wherein the control unit determines that whether or not reflected light exists in the eye image when the control unit analyzes the images of the eye obtained by the capturing unit; and
wherein the control unit selects the eye image of the person to be authenticated in which the reflected light of illumination light emitted from the illuminating device is not superposed on the eye image.

10. The eye image capturing apparatus as set forth in claim 4, wherein when reflected light exists in the eye images, the control unit extracts a plurality of partial eye images where the reflected light does not exist from the eye images; and
wherein the control unit synthetically incorporates the partial eye images into an eye image in which the reflected light does not exist.
